(19) Europäisches Patentamt
European Patent Office
Office européen des brevets

(11) EP 0 711 141 B1

(12) **FASCICULE DE BREVET EUROPEEN**

(45) Date de publication et mention de la délivrance du brevet:
**11.06.1997 Bulletin 1997/24**

(21) Numéro de dépôt: **94923759.8**

(22) Date de dépôt: **28.07.1994**

(51) Int. Cl.$^6$: **A61K 7/48**, A61K 35/78

(86) Numéro de dépôt international:
**PCT/FR94/00956**

(87) Numéro de publication internationale:
**WO 95/03780 (09.02.1995 Gazette 1995/07)**

(54) **COMPOSITION COSMETIQUE OU PHARMACEUTIQUE, NOTAMMENT DERMATOLOGIQUE, CONTENANT UN EXTRAIT DE TEPHROSIA, EN PARTICULIER TEPHROSIA PURPUREA**

TEPHROSIA-EXTRAKT, INSBESONDERE TEPHROSIA PURPUREA ENTHALTENDE KOSMETISCHE ODER PHARMAZEUTISCHE, INSBESONDERE DERMATOLOGISCHE, ZUSAMMENSETZUNG

COSMETIC OR PHARMACEUTICAL AND PARTICULARLY DERMATOLOGICAL COMPOSITION CONTAINING AN EXTRACT OF TEPHROSIA, PARTICULARLY TEPHROSIA PURPUREA

(84) Etats contractants désignés:
**BE CH DE ES FR GB IT LI**

(30) Priorité: **28.07.1993 FR 9309283**

(43) Date de publication de la demande:
**15.05.1996 Bulletin 1996/20**

(73) Titulaire: **PARFUMS CHRISTIAN DIOR**
**75008 Paris (FR)**

(72) Inventeurs:
- **ANDRE, Patrice**
  **F-45170 Neuvilles-aux-Bois (FR)**
- **DARNAULT, Sylvie**
  **F-45000 Orléans (FR)**
- **RENIMEL, Isabelle**
  **F-45470 Trainou (FR)**

(74) Mandataire: **Portal, Gérard et al**
**Cabinet Beau de Loménie**
**158, rue de l'Université**
**75340 Paris Cédex 07 (FR)**

(56) Documents cités:
**GB-A- 2 237 805**

- **DATABASE WPI Week 8519, Derwent Publications Ltd., London, GB; AN 85-113075 & JP,A,60 054 312 (NISSAN CHEM IND KK ET AL) 28 Mars 1985**
- **DATABASE BIOSIS BIOSCIENCES INFORMATION SERVICE, PHILADELPHIA, PA, US AN: 85:428264 H. RAHMAN ET AL 'Hypoglycemic activity of Tephrosia purpurea seeds' cité dans la demande**
- **DATABASE BIOSIS BIOSCIENCES INFORMATION SERVICE, PHILADELPHIA, PA, US AN: 93:323684 M. S. R. MURTHY ET AL 'Hepatoprotective effect of Tephrosia Purpurea in experimental animals'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 711 141 B1

## Description

La présente invention concerne essentiellement l'utilisation d'un extrait de Tephrosia, en particulier Tephrosia purpurea pour la préparation d'une composition pharmaceutique, notamment dermatologique, ou comme agent cosmétique, et un procédé de traitement cosmétique en comportant application.

Les plantes du genre Tephrosia sont des plantes subtropicales largement distribuées notamment en Inde ou au Sri Lanka.

Une espèce particulièrement préférée dans le cadre de l'invention dans le genre Tephrosia est la plante de l'espèce Tephrosia purpurea. C'est une plante perenne de 30 à 100 cm de haut donnant par gousse 4 à 6 graines et ayant des fleurs rouges. L'espèce Tephrosia purpurea est surtout connue en Inde et est essentiellement connue en médecine Ayurvedique, c'est-à-dire à médecine traditionnelle indienne. Différentes parties ont été utilisées pour le traitement de diverses maladies, bronchites, maladies du foie et du rein, purification du sang (voir article de Pandey dans Quart. J. Crude Drug Res. 13, (1975), pages 65-68). Les graines de Tephrosia purpurea ont également été utilisées dans le cas d'une activité hypoglycémique (voir article de Rahman et al. dans Indian J. Med. Res. 81, avril 1985, pages 418-421). On connaît encore l'activité antimicrobienne in vitro de Tephrosia purpurea (voir article de Agar'val et al. dans Indian J. Physiol. Pharmac. vol. 31, n° 4, d'octobre-décembre 1987, pages 284-286), à partir d'extrait de racine à l'alcool à 90 % dans un appareil de Soxhlet.

Après des recherches intensives sur la plante du genre Tephrosia, et en particulier de l'espèce Tephrosia purpurea, les présents inventeurs ont découvert de manière inattendue que des extraits, en particulier de graines de plante Tephrosia, notamment de Tephrosia purpurea, présentent une activité de stimulation puissante de l'enzyme adénylate cyclase. On sait que cette enzyme transforme l'ATP en AMPc et pyrophosphate. Grâce à cette activité, les extraits du genre Tephrosia, en particulier de l'espèce Tephrosia purpurea, sont précieux pour une application en cosmétique ou en pharmacie, notamment en dermatologie, dans une activité d'amincissement corporel, dans une activité anti-inflammatoire, dans une activité pigmentante par action sur les mélanocytes et une activité antivieillissement.

Ainsi, selon un premier aspect, la présente invention couvre l'utilisation d'un extrait de la plante du genre Tephrosia, en particulier de l'espèce Tephrosia purpurea, comme substance active pour la préparation d'une composition pharmaceutique, notamment dermatologique, ayant une activité de stimulation de l'enzyme adénylate cyclase, une activité amincissante, une activité anti-inflammatoire, une activité pigmentante et une activité anti-vieillissement, en particulier par voie topique, éventuellement dans un support, véhicule ou excipient pharmaceutiquement, notamment dermatologiquement, acceptable.

L'invention couvre également l'utilisation de l'extrait de la plante du genre Tephrosia, en particulier l'espèce Tephrosia purpurea, comme agent cosmétique pour stimuler l'enzyme adénylate cyclase, pour obtenir un effet d'amincissement corporel, pour obtenir un effet pigmentant ou pour obtenir un effet antivieillissement, en particulier par voie topique.

Selon un mode de réalisation avantageux, l'extrait précité est un extrait de graines de la plante Tephrosia, en particulier un extrait de graine de l'espèce Tephrosia purpurea.

Il est à noter que la plante Tephrosia purpurea est également connue, notamment d'après la base de données NAPRALERT® sous les noms synonymes suivants : Cracca purpurea, Galeduba lanceaefolia, Galeduba purpurea, Galepa tinctoria, Tephrosia galegoïdes, Tephrosia indigofera, Tephrosia lobata, Tephrosia maxima, qui sont naturellement couverts par la présente invention.

Selon un mode de réalisation avantageux, cet extrait de graines est un extrait hydroalcoolique avec un alcool en $C_1$-$C_6$ linéaire ou ramifié cyclique. Un alcool particulièrement préféré est le méthanol ou l'éthanol. Les proportions relatives eau-alcool peuvent varier dans de larges limites. On préfère cependant les proportions relatives en volume sensiblement égales.

Selon un deuxième aspect, la présente invention couvre également un procédé de traitement cosmétique pour obtenir une stimulation de l'enzyme adénylate cyclase, pour obtenir un effet d'amincissement corporel, pour obtenir un effet pigmentant ou un effet anti-vieillissement, caractérisé en ce qu'il comprend l'application par voie topique sur des zones concernées d'un patient, en particulier l'épiderme ou les cheveux, d'une quantité cosmétiquement efficace d'un extrait de la plante du genre Tephrosia, en particulier de l'espèce Tephrosia purpurea, éventuellement dans un excipient, support ou véhicule cosmétiquement acceptable.

Dans l'un quelconque des aspects précédents, selon un mode de réalisation avantageux, on utilisera de 0,001 à 5 %, de préférence 0,01 à 5 %, en poids d'extrait par rapport au poids total de la composition.

D'autres buts, avantages de l'invention apparaîtront clairement à la lumière de la description explicative qui va suivre faite en référence à divers exemples de réalisation de l'invention donnés simplement à titre d'illustration et qui ne sauraient donc en aucune façon limiter la portée de l'invention. Dans la description et les revendications, sauf indication contraire, les pourcentages sont donnés en poids.

**EXEMPLE 1**

Fabrication de l'extrait de la plante de l'extrait Tephrosia purpurea

On prend 1000 g de graines de Tephrosia purpurea achetées dans le commerce que l'on broie et tamise à 1 mm. On laisse macérer 24 h dans 5 l de solvant d'extraction hydroalcoolique constitué ici par un mélange méthanol-eau à 50/50 V/V.

On porte ensuite à ébullition sous reflux pendant 1 h.

On filtre ensuite sur papier, éventuellement sous pression. On lave au méthanol froid.

Le filtrat est ensuite évaporé sous vide jusqu'à disparition totale de l'alcool et de l'eau.

On pèse le résidu, soit 80,11 g d'extrait d'aspect hétérogène comprenant un cristalloïde clair et une pâte foncée.

Cet extrait brut contient environ 50 % de matières sèches et 50 % de solvants résiduels.

Il peut être davantage purifié selon les méthodes classiques bien connues à l'homme de l'art ou être utilisé tel quel.

**EXEMPLE 2**

On prend 2,365 kg de graines de la plante Tephrosia purpurea disponible dans le commerce que l'on broie et tamise à 1 mm puis que l'on dégraisse avec de l'éther de pétrole.

Les graines broyées dégraissées sont ensuite extraites dans un appareil de Soxhlet d'environ 10 l d'éthanol pendant 2 h.

Après évaporation du solvant, on obtient un résidu se présentant sous forme d'une gomme brunâtre pesant environ 58 g.

**EXEMPLE 3**

Utilisation d'extrait de Tephrosia purpurea pour mettre en évidence la stimulation de l'activité adénylate cyclase.

Certaines substances servent de messager intracellulaire et, de par ce fait, sont des constituants cellulaires essentiels qui adaptent le fonctionnement des cellules aux besoins de l'organisme. Parmi ces messagers, l'on trouve l'adénosine 3',5' qui est un monophosphate cyclique (AMPc). Le métabolisme de cette molécule se fait à partir de l'adénosine 3',5'-triphosphate grâce à une enzyme appelée l'adénylate cyclase.

Le principe réactionnel est le suivant :

$$\text{ATP} \xrightarrow{\frac{\text{Adénylate}}{\text{cyclase}}} \text{AMPc + pyrophosphate}$$

Principe

On obtient d'abord des membranes cellulaires à partir de culture à confluence de fibroblastes de la lignée 3T3 F442A disponible dans le commerce auprès de la société Flow Laboratories.

Ces membranes cellulaires de fibroblastes, constituant une source d'enzyme, sont incubées dans le milieu réactionnel en présence d'ATP à 37°C pendant un temps préalablement défini.

L'AMPc formé est dosé par Radio Immuno Assay. En considérant l'activité basale, c'est-à-dire sans effecteur, comme étant de 100 %, on pourra déterminer l'influence d'un effecteur sur le système enzymatique. Si l'activité est supérieure à 100 %, il sera mis en évidence un effet activateur de l'adénylate cyclase et, si l'activité est inférieure à 100 %, il sera mis en évidence au contraire un effet inhibiteur.

Mise en oeuvre

Les membranes cellulaires sont purifiées à partir de fibroblaste en culture.

A ce propos, il faut observer que la purification des membranes cellulaires libère de nombreuses enzymes dans le milieu de dosage. Parmi celles-ci, deux sont indésirables pour la mise en oeuvre du test. Il s'agit d'une part de la $Na^+$, $K^+$ ATPase qui transforme l'ATP en adénosine diphosphate, et d'autre part de la phosphodiestérase 3',5'-AMPc qui dégrade l'AMP cyclique en AMP. Aussi est-il indispensable, pour obtenir des résultats fiables de ce test, d'inhiber ces deux enzymes. La première le sera au moyen de l'ouabaïne, et la seconde au moyen de la théophylline.

Le milieu réactionnel est donc constitué de :

- 50 µl d'une solution 5 mM d'adénosine 3',5'-triphosphate (ATP) à titre de substrat
- 100 µl d'une solution 20 mM d'ouabaïne comme inhibiteur de l'ATPase
- 100 µl d'une solution 20 mM de Théophilline comme inhibiteur de phosphodiestérase

- 100 µl d'une solution à 0,05 g/l en matière sèche de l'effecteur

Après incubation, on arrête la réaction par chauffage pendant 10 min à 100°C, on centrifuge et l'on récupère le sumageant pour doser l'AMPc par Radio Immuno Assay (Kit Immunotech, société française, référence 1117).

Résultats

Les résultats des essais sont répertoriés dans le tableau ci-après :

TABLEAU 1

| | Activité basale | Témoin positif= forskoline à 0,05 g/l | Tephrosia purpurea à 0,05 g/l en extrait sec de l'exemple 2 |
|---|---|---|---|
| % d'activité | 100 | 225 | 185 |

Il ressort très clairement des résultats ci-dessus que l'extrait de la plante Tephrosia purpurea possède une activité significative importante de stimulation de l'enzyme adénylate cyclase. En effet, cette activité est relativement proche de celle du témoin positif constitué par la forskoline, bien que l'extrait de cette plante ne contient pas cette molécule.

Ainsi, les extraits de Tephrosia, en particulier de Tephrosia purpurea constituent une alternative intéressante à la forskoline dans son utilisation pour la préparation de compositions cosmétiques ou pharmaceutiques.

Les applications de la présente invention sont notamment celles qui découlent des processus biologiques impliquant le rôle de l'AMPc. Parmi celles-ci, les applications préférées de l'invention sont la préparation de composition amincissantes, de compositions pigmentantes pour la peau ou les cheveux, de compositions anti-inflammatoires et de compositions destinées à lutter contre les effets du vieillissement.

**EXEMPLE 4**

Mise en évidence de l'activité lipolytique des compositions selon l'invention

- Evaluation de l'action des compositions selon l'invention sur les adipocytes en culture

Il a été choisi d'évaluer l'efficacité des compositions selon l'invention en tant qu'agents lipolytiques sur une lignée de pré-adipocytes murins, telle qu'une lignée 3T3 F442A disponible dans le commerce auprès de la Société Flow Laboratories, qui ont été sélectionnés pour leur capacité à se convertir en adipocytes si les conditions de culture le permettent.

(Conformément à la méthode de Green, H & Kehinde C Cell 1 (1974) 113).

Cette lignée constitue en effet un modèle d'étude de la différenciation adipocytaire in vitro tout en offrant la possibilité lorsque le phénotype adipocytaire est atteint d'étudier les régulations du fonctionnement cellulaire selon l'invention extra-cellulaire. Cette différenciation et sa modulation s'accompagnent d'un certain nombre de modifications morphologiques et biochimiques et en particulier, les modifications biochimiques concernent la libération du glycérol, au cours de la lipolyse.

Il est donc commode de vérifier l'efficacité des composés à tester par une mesure de l'activité de l'enzyme glycérol-3-phosphate déshydrogénase (dénommé G3PDH) qui intervient dans la réaction de lipogénèse suivante :

$$\text{dihydroxyacétone phosphate (DHAP)} + \text{NADH}, H^+ \xrightarrow{\text{G3PDH}} \text{glycérol-3-phosphate} + \text{NADH}$$

Ainsi, l'activité enzymatique peut être mesurée selon la méthode de dosage du NADH libéré par la méthode de dosage décrite par Pairault J. et al. dans Proc. Nat. Acad. Sci., volume 6, (1979), pages 5138-42.

Ces expérimentations sont réalisées comme suit :

1) Conditions de culture

Les pré-adipocytes sont ensemencés dans des boîtes de Pétri de 35 mm de diamètre (20 000 cellules/boite), en présence de DMEM ("Dulbecco's Modified Eagle Medium") contenant 5 % de sérum de veau plus 5 % de sérum de veau foetal.

Le milieu est renouvelé deux fois par du milieu DMEM additionné de 10 % de sérum de veau foetal.

Dans ces conditions, la culture atteint la confluence en une semaine (J = Jo), à ce stade, la différenciation adipocytaire est activée par l'addition d'insuline à la concentration de 1 % en poids du milieu de culture. On procède également à deux changements de milieu de culture DMEM addtionnés à 10 % de sérum de veau foetal et de 1 % d'insuline.

Ces cellules présentent un état différencié avancé une semaine après la confluence ($J = J_7$).

2) Traitement - Viabilité

Les cellules sont traitées par les produits à tester au stade $J_7$.

Ce traitement consiste à remplacer le milieu de culture soit par du milieu complet pour le témoin, soit par ce même milieu contenant le produit à tester à différentes concentrations.

Pour le dosage d'activité de G3PDH, le milieu de culture est récupéré 7 jours (J = J14) après le traitement.

3) Dosage au jour J14 de l'activité enzymatique G3PDH

La monocouche cellulaire est récupérée par grattage et vigoureusement homogénéisée dans du tampon TRIS HCl 25 mM, pH 7,4, 1 mM EDTA à 4°C. Le dosage de l'activité G3PDH est déterminé sur le surnageant du broyat cellulaire après centrifugation.

Ce dosage, effectué selon la méthode de dosage de Pairault précédemment citée, mesure l'activité enzymatique, G3PDH rapportée au milligramme de protéine. La teneur est protéine est déterminée par la méthode de dosage de protéine de Lowry J. décrite dans Biol. Chem. 193, (1951), pages 265-75, cette activité enzymatique est exprimée en nanomole/min/mg de protéine.

On effectue trois essais par échantillon ou par témoin.

Les produits testés sont les suivants :

a) un premier produit témoin pour lequel le milieu DMEM additionné de 7,5 µl d'éthanol pour 6 ml de milieu de culture,

b) le même milieu mais additionné de 60 µl de DMSO (diméthylsulfoxyde) pour 6 ml de milieu de culture,

c) le même milieu mais additionné d'un extrait de Coleus forskohlii, contenant de la forskoline, disponible dans le commerce, à une concentration de 0,025 g/l exprimée en poids sec, dilué dans 7,5 µ/l d'éthanol pour 6 ml de milieu de culture,

d) un produit dans lequel le milieu DMEM précité a été additionné de 0,2 g/l en extrait sec de Tephrosia purpurea tel qu'obtenu à l'exemple 2, dilué dans 60 µl de DMSO pour 6 ml de milieu de culture,

e) un produit selon lequel le milieu DMEM a été additionné de 0,1 g/l d'extrait sec de Tephrosia purpurea obtenu à l'exemple 2, dilué dans 60 µl de DMSO pour 6 ml de milieu de culture,

f) un produit dans lequel le milieu DMEM a été additionné de 0,05 g/l d'extrait sec de Tephrosia purpurea obtenu à l'exemple 2, dilué dans 60 µl de DMSO pour 6 ml de milieu de culture.

Les résultats obtenus sont répertoriés au tableau 2 suivant :

TABLEAU 2

| Produit testé | Activité G3PDH exprimée en nanomole de NADH libéré par min/ml de protéine |
|---|---|
| Témoin éthanol | 382,68 |
| Témoin DMSO | 392,36 |
| Coleus forskohlii | 176,48 |
| Tephrosia (0,2 g/l) | 155,23 |
| Tephrosia (0,1 g/l) | 353,03 |
| Tephrosia (0,05 g/l) | 369,64 |

Il ressort très clairement des résultats du tableau 2 ci-dessus que l'extrait de la plante Tephrosia purpurea possède une activité significative importante d'inhibition de l'activité G3PDH, traduite par une faible quantité de NADH libéré. En outre, à partir d'une concentration de 0,02 g/l, cette activité est similaire ou légèrement supérieure à l'activité obtenue avec l'extrait de Coleus forskohlii qui contient de la forskoline, connue pour son activité de stimulation de l'adénylate cyclase et son effet sur les lipolyses.

Or, il peut être avantageux d'inhiber l'activité G3PDH, avec un produit qui ne contient pas de forskoline en constituant ainsi une alternative intéressante à la forskoline dans son utilisation pour la préparation de compositions cosmétique ou pharmaceutique, pour les activités qui ont été cités précédemment.

La présente invention sera maintenant décrite en référence à divers exemples de formulations de composition cosmétique ou pharmaceutique, notamment dermatologique.

**EXEMPLE 5**

Crème corporelle amincissante

| | |
|---|---|
| - Extrait de graines de Tephrosia purpurea selon l'exemple 1 | 1 % |
| - Excipient pour crème q.s.q | 100 % |

Cette crème est appliquée au moins une fois par jour pendant 2 à 3 semaines sur les zones du corps comportant des substances graisseuses jusqu'à obtention de l'effet d'amincissement recherché.

**EXEMPLE 6**

Composition cosmétique pigmentante

Cette composition comprend les constituants suivants :

| | |
|---|---|
| - Extrait de graines de Tephrosia purpurea selon l'exemple 1 | 0,5 % |
| - Excipient gel cosmétique habituel q.s.p | 100 % |

Ce gel est appliqué sur les zones de la peau à pigmenter jusqu'à obtention de l'effet désiré.

**EXEMPLE 7**

Composition pharmaceutique, notamment dermatologique, anti-inflammatoire

Cette composition comprend les constituants suivants :

| | |
|---|---|
| - Extrait de graines de Tephrosia purpurea selon l'exemple 1 | 0,2 % |
| - Excipient pharmaceutiquement acceptable q.s.p | 100 % |

**EXEMPLE 8**

Composition cosmétique antivieillissement

Cette composition comprend les constituants suivants :

| | |
|---|---|
| - Extrait de graines de Tephrosia purpurea selon l'exemple 1 | 0,1 % |
| - Excipient cosmétique habituel q.s.p | 100 % |

Cette composition est appliquée sur les zones de la peau où un effet antivieillissement est recherché pendant une période de temps suffisante, en général de l'ordre de plusieurs semaines à plusieurs mois selon les sujets.

**Revendications**

1. Utilisation d'un extrait de la plante du genre Tephrosia, en particulier de l'espèce Tephrosia purpurea, comme substance active, pour la préparation d'une composition pharmaceutique, notamment dermatologique, ayant une activité de stimulation de l'enzyme adénylate cyclase, une activité d'amincissement corporel, une activité anti-inflammatoire, une activité pigmentante et une activité antivieillissement, en particulier par voie topique, éventuellement dans un support, véhicule ou excipient pharmaceutiquement, notamment dermatologiquement, acceptable.

2. Utilisation d'un extrait de la plante du genre Tephrosia, en particulier de l'espèce Tephrosia purpurea, comme agent cosmétique pour stimuler l'enzyme adénylate cyclase, pour obtenir un effet d'amincissement corporel, pour obtenir un effet pigmentant ou pour obtenir un effet anti-vieillissement, en particulier par voie topique.

3. Utilisation selon la revendication 1 ou 2, caractérisée en ce que l'extrait précité est un extrait de graines.

4. Utilisation selon la revendication 1 ou 3, caractérisée en ce que l'extrait précité est un extrait de graines de l'espèce Tephrosia purpurea.

5. Utilisation selon l'une des revendications précédentes, caractérisée en ce que l'extrait est présent dans une composition à raison de 0,001 à 5 %, de préférence 0,01 à 5 %, en poids d'extrait de Tephrosia par rapport au poids total de la composition.

6. Utilisation selon l'une quelconque des revendications précédentes, caractérisée en ce que l'extrait précité est un extrait obtenu à partir d'un solvant d'extraction alcoolique en $C_1$-$C_6$ ou hydro-alcoolique en $C_1$-$C_6$ ; le solvant alcoolique est en particulier choisi parmi le méthanol ou l'éthanol.

7. Procédé de traitement cosmétique pour obtenir une stimulation de l'enzyme adénylate cyclase, pour obtenir un effet d'amincissement corporel, pour obtenir un effet pigmentant ou un effet anti-vieillissement, caractérisé en ce qu'il comprend l'application par voie topique sur des zones concernées d'un patient, en particulier l'épiderme ou les cheveux d'une quantité cosmétiquement efficace d'un extrait de la plante du genre Tephrosia, en particulier de l'espèce Tephrosia purpurea, éventuellement dans un excipient, support ou véhicule cosmétiquement acceptable.

8. Procédé selon la revendication 7, caractérisé en ce qu'on applique l'extrait sous forme d'une composition qui comprend de 0,001 à 5 %, de préférence 0,01 à 5 %, en poids d'extrait par rapport au poids total de la composition.

9. Procédé selon la revendication 7 ou 8, caractérisé en ce qu'on applique l'extrait de Tephrosia sur des zones de l'épiderme où un amincissement corporel est recherché.

10. Procédé selon la revendication 7 ou 8, caractérisé en ce qu'on applique l'extrait de Tephrosia sur des zones de l'épiderme ou des cheveux où une pigmentation est recherchée.

11. Procédé selon la revendication 7 ou 8, caractérisé en ce que l'extrait de Tephrosia est appliqué sur des zones de l'épiderme où une activité antivieillissement est recherchée.

**Claims**

1. A use of an extract of a plant of the genus Tephrosia, in particular the species Tephrosia purpurea, as an active substance for preparing a pharmaceutical composition, in particular a dermatological composition, presenting activity in stimulating the enzyme adenylate cyclase, body-slimming activity, anti-inflammatory activity, pigmenting activity, and anti-aging activity, in particular when topically applied, optionally in a carrier, vehicle, or excipient that is pharmaceutically and in particular dermatologically acceptable.

2. The use of an extract of a plant of the genus Tephrosia, in particular of the species Tephrosia purpurea, as a cosmetic agent for stimulating the enzyme adenylate cyclase, to obtain a body-slimming effect, to obtain a pigmenting effect, or to obtain an anti-aging effect, in particular when topically applied.

3. The use according to claim 1 or 2, characterized in that the aforesaid extract is a seed extract.

4. The use according to claim 1 or 3, characterized in that the aforesaid extract is a seed extract of the species Tephrosia purpurea.

5. The use according to one of the preceding claims, characterized in that the extract is present in the composition at a concentration in the range 0.001% to 5%, preferably 0.01% to 5% by weight of Tephrosia extract relative to the total weight of the composition.

6. The use according to any one of the preceding claims, characterized in that the aforesaid extract is an extract obtained from a $C_1$-$C_6$ alcohol or $C_1$-$C_6$ hydroalcohol extraction solvent; the alcohol solvent being selected in particular from methanol and ethanol.

7. A method of cosmetic treatment for obtaining stimulation of the enzyme adenylate cyclase to obtain a body-slimming effect, to obtain a pigmenting effect, or an anti-aging effect, characterized in that it comprises topical application on the areas concerned of a patient, in particular the epidermis or the hair, of a cosmetically effective quantity of an extract of a plant of the genus Tephrosia, in particular the species Tephrosia purpurea, optionally in a cosmetically acceptable excipient, carrier, or vehicle.

8. The method according to claim 7, characterized in that the extract is applied in the form of a composition containing 0.001% to 5% and preferably 0.01% to 5% by weight of extract relative to the total weight of the composition.

9. The method according to claim 7 or 8, characterized in that the Tephrosia extract is applied on areas of the epidermis where body-slimming is sought after.

10. The method according to claim 7 or 8, characterized in that the Tephrosia extract is applied to areas of the epidermis or of the hair where pigmentation is sought after.

11. The method according to claim 7 or 8, characterized in that the Tephrosia extract is applied on areas of the epidermis where anti-aging activity is sought after.

**Patentansprüche**

1. Verwendung eines Extrakts der Pflanze der Gattung Tephrosia, insbesondere der Art Tephrosia purpurea, als aktive Substanz bei der Herstellung einer pharmazeutischen, insbesondere dermatologischen Zusammensetzung

mit einer Wirksamkeit zur Stimulation des Enzyms Adenylatcyclase, einer körperlich schlankmachenden Wirksamkeit, einer entzündungshemmenden Wirksamkeit, einer pigmentierenden Wirksamkeit und einer Anti-Alterungs-Wirksamkeit, spezifisch auf topischem Weg, gegebenenfalls in einem pharmazeutisch, insbesondere dermatologisch annehmbaren Träger, Vehikel oder Exzipienten.

**2.** Verwendung eines Extrakts der Pflanze der Gattung Tephrosia, insbesondere der Art Tephrosia purpurea, als kosmetisches Mittel zur Stimulation des Enzyms Adenylatcyclase, um eine körperlich schlankmachende Wirkung zu erhalten, um eine pigmentierende Wirkung zu erhalten, oder um eine Anti-Alterungs-Wirkung zu erhalten, spezifisch auf topischem Weg.

**3.** Verwendung nach Anspruch 1 oder 2, dadurch gekennzeichnet, daß der Extrakt ein Korn-Extrakt ist.

**4.** Verwendung nach Anspruch 1 oder 3, dadurch gekennzeichnet, daß der Extrakt ein Korn-Extrakt der Art Tephrosia purpurea ist.

**5.** Verwendung nach einem der vorhergehenden Ansprüche, dadurch gekennzeichnet, daß der Extrakt in einer Zusammensetzung bei 0,001 bis 5 Masse-%, vorzugsweise 0,01 bis 5 Masse-% Tephrosia-Extrakt, bezogen auf die Gesamtmasse der Zusammensetzung, vorliegt.

**6.** Verwendung nach einem der vorhergehenden Ansprüche, dadurch gekennzeichnet, daß der Extrakt ein Extrakt ist, der aus einem alkoholischen $C_1$-$C_6$- oder hydroalkoholischen $C_1$-$C_6$-Extraktionslösungsmittel erhalten wird; wobei das alkoholische Lösungsmittel insbesondere aus Methanol oder Ethanol ausgewählt wird.

**7.** Verfahren zur kosmetischen Behandlung, um eine Stimulation des Enzyms Adenylatcyclase zu erhalten, um eine körperlich schlankmachende Wirkung zu erhalten, um eine pigmentierende Wirkung oder eine Anti-Alterungs-Wirkung zu erhalten, dadurch gekennzeichnet, daß es das Aufbringen einer kosmetisch wirksamen Menge eines Extrakts der Pflanze der Gattung Tephrosia, insbesondere der Art Tephrosia purpurea, gegebenenfalls in einem kosmetisch annehmbaren Exzipienten, Träger oder Vehikel, auf topischem Weg auf den betreffenden Zonen eines Patienten, insbesondere die Epidermis oder die Haare umfaßt.

**8.** Verfahren nach Anspruch 7, dadurch gekennzeichnet, daß der Extrakt in Form einer Zusammensetzung, die 0,001 bis 5 Masse-%, vorzugsweise 0,01 bis 5 Masse-% Extrakt, bezogen auf die Gesamtmasse der Zusammensetzung, aufgebracht wird.

**9.** Verfahren nach Anspruch 7 oder 8, dadurch gekennzeichnet, daß der Tephrosia-Extrakt auf den Zonen der Epidermis, wo eine körperlich schlankmachende Wirkung erwünscht ist, aufgebracht wird.

**10.** Verfahren nach Anspruch 7 oder 8, dadurch gekennzeichnet, daß der Tephrosia-Extrakt auf den Zonen der Epidermis oder der Haare, wo eine Pigmentation erwünscht ist, aufgebracht wird.

**11.** Verfahren nach Anspruch 7 oder 8, dadurch gekennzeichnet, daß der Tephrosia-Extrakt auf den Zonen der Epidermis, wo eine Anti-Alterungs-Wirksamkeit erwünscht ist, aufgebracht wird.